(19) Europäisches Patentamt
European Patent Office
Office européen des brevets

(11) Publication number: 0 270 595 B1

(12) **EUROPEAN PATENT SPECIFICATION**

(45) Date of publication of patent specification: **16.08.90**

(51) Int. Cl.$^5$: **C 07 B 57/00**, C 07 C 69/96, C 07 D 207/404

(21) Application number: **87903467.6**

(22) Date of filing: **18.05.87**

(86) International application number: **PCT/SE87/00245**

(87) International publication number: **WO 87/06929 19.11.87 Gazette 87/25**

# (54) AN OPTICALLY ACTIVE REAGENT AND A METHOD FOR THE DETERMINATION OF ENANTIOMERIC AMINE COMPOUNDS.

(30) Priority: **16.05.86 US 863712**

(43) Date of publication of application: **15.06.88 Bulletin 88/24**

(45) Publication of the grant of the patent: **16.08.90 Bulletin 90/33**

(84) Designated Contracting States: **AT BE CH DE FR GB IT LI NL SE**

(56) References cited:

**Chemical Abstracts vol. 88, 1978, 88:146083t Resolution of antimalarial agents via complex formation with alpha-(2,4,5,7-tetranitro-9-fluorenylideneami nooxy) propionic acid.**
**J.Med. Chem. 1978, 21(4), 326-330**
**J. Liqu. Chromatography, 1979, 2, 1229-1250**

**The file contains technical information submitted after the application was filed and not included in this specification**

(73) Proprietor: **EKA NOBEL AB**
**Chem. Intermediates Division**
**S-445 01 Surte (SE)**

(72) Inventor: **JOSEFSSON, Björn**
**Rangströmsliden 3**
**S-413 18 Göteborg (SE)**
Inventor: **EINARSSON, Stefan**
**Bergsgatan 12**
**S-413 01 Göteborg (SE)**
Inventor: **SANCHEZ, Domingo**
**Snipasvagen 24**
**S-448 00 Floda (SE)**
Inventor: **MÖLLER, Per**
**Svensksundsgatan 1 D**
**S-416 61 Göteborg (SE)**

(74) Representative: **Schöld, Zaid**
**Nobel Industries Sweden AB Patent Department**
**Box 11554**
**S-100 61 Stockholm (SE)**

**EP 0 270 595 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the Invention

This invention relates to an improvement in enantiomeric separation and detection by means of a chiral reagent and high performance liquid chromatography (HPLC). More specifically it relates to an optically active fluorenyl chloroformate which forms stable diastereomeric carbamates with primary and secondary amines in a quantitative way. This product can be detected by fluorimetry or by absorptiometry after separation on HPLC column.

The separation of optically active amino-containing substances is of great importance in biological research and pharmaceutical chemistry. These substances are common in pharmaceuticals and many of them are enantiomers occurring as racemic mixtures. In many cases the biological activity of these enantiomers is attributed to the conformation of one particular optically active antipode. Therefore it is very important to distinguish or resolve the enantiomers.

For quantitive purpose chromatographic methods are to be used since they offer many advantages such as small sample size, simple pretreatment and high sensitivity of detection.

Since the first chromatographic separation of optically active isomers in 1951 with paper chromatography technique a great number of such methods have been introduced and they have recently been reviewed in books (Souter, R. W. "Chromatographic Separation of Stereoisomers"; CRC Press, Boca Raton, 1985 and Jacques, J.; Collet, A.; Wilen, S. H. "Enantiomers, Racemates and Resolutions"; John Wiley & Sons; New York, 1981).

The resolution of enantiomers requires the intervention of a chiral agent. Chromatographic resolving methods have been developed in two major directions:

1. Direct resolution of enantiomers in columns with either a chiral stationary phase (CSP) or a chiral component in the mobile phase.

2. Indirect resolution of formed diastereomers after derivatization with a chiral reagent.

The liquid chromatographic direct separation of enantiomers on chiral stationary phases has received a great deal of attention and nowadays there are some commercially available chiral columns. The efforts have been focused on to make a chiral stationary phase feasible to separate as many different types of chiral substances as possible. Still there are very few amines and amino acids that have been directly resolved on chiral columns of any type, without previous derivatization.

Tamegai et al. J. Liq. Chromatography, 1979, 2, 1229 have reviewed methods based on indirect resolution of enantiomers by derivatization with a chiral reagent to form diastereometers which are resolved on conventional columns. Furukawa et al, Chem. Pharm. Bull. 1975, 23, 1623 were the first to separate enantiomeric amino acids using readily available (+)-10-camphor sulphonyl chloride as a chiral reagent. The carbonyl residue was then transformed in a second step into the p-nitrobenzylester which resulted in derivatives sensitive to UV-detection.

The most common chiral reagents for selective amino function derivitization are those based on isothiocyanate which give UV sensitive thiourea derivatives with primary and secondary amines. The reaction is effected under weakly basic conditions for about 20 minutes at room temperature without formation of by-products.

The most successful reagent of this kind is 2,3,4,6-tetra-o-acetyl-p-d-gluco-pyranosyl isothiocyante (GITC) used for both amino acids and amines. The resulting thiourea derivatives of most protein amino acids were resolved on a conventional reversed phase column within two hours. The resolution obtained is preferably due to the lipophilic nature of the sugar residue combined with conformational rigidity.

A specific fluorenyl based compound has been used for resolution of amines. This is disclosed in J. Med. Chem. 1978, 21(4), 326—330 which reports the resolution of antimalarial agents via complex formation with α-(2,4,5,7-tetranitro-9-fluorenylideneaminooxy)propionic acid (TAPA). The resolution is carried out by fractional crystallization of the TAPA-complexes.

Fluorescence chiral derivatization reagents for amino containing compounds are rare and only reagents based on o-phtalaldehyde combined with chiral thiols (OPA) are reported, JP 60 38,652 (85 38,652). The different chiral thiol compounds which have been used are N-acetyl-L-cysteine and BOC-L-cysteine and these yield diastereomeric isoindols with primary amino groups. The reaction is selective and completed within a minute at room temperature direct in buffered water mixture without racemization. The formed diastereomers are however not stable.

There are some essential and desirable conditions such a reagent should have:

* It has to be optically pure.
* It has to contain a chromophore or a fluorophore.
* The reaction conditions should be mild, otherwise the risk of potential racemization will increase.
* The reagent should react with primary and secondary amino groups.
* The resulting diastereomers should be stable.
* The resulting diastereomers should separate on conventional HPLC columns.
* The method should be applicable on a preparative scale.
* The method should be feasible to automatize.

Nowhere, there has however been disclosed or suggested a chiral reagent combined with a method which effectively derivatize, detect and separate racemic mixtures of primary and secondary amino-containing compounds.

## Summary of the Invention

The object of the present invention is to provide a chiral reagent and a method for indirect resolution of primary and secondary amino-containing compounds on conventional reversed phase HPLC columns. The reagent should give fluorescent derivatives which are stable, and detectable by fluorometry or absorptiometry after separation by liquid chromatography. A stable derivative facilitates quantitation and automation. Furthermore the reaction should occur rapidly under mild conditions in aqueous phase and without racemization.

This has been achieved by derivatizing the amino function of said compounds by adding an optically active reagent of the formula

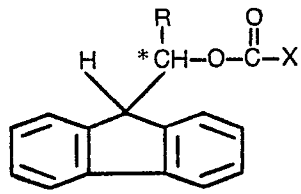

wherein X is a halogen, an azide group or a succinimidyl group and wherein R is an alkyl group or a trifluoromethyl group, to form diastereomeric carbamates which are determined by per se known methods, e.g. fluorimetry or absorptiometry, after separation by liquid chromatography.

The reagent performs:

1. The determination of optical purity in resolved amines and amino acids.
2. The determination of different amounts of enantiomeric amino-containing compounds in biological systems.
3. Preparative resolution of amino-containing racemic mixtures.

## Description of the drawings

Fig. 1 shows a reagent and reaction scheme.

Fig. 2 is a graph showing reaction rates and yields to form fluorescent diastereomatic carbamates of different amino acids.

Fig. 3 is a graph showing reaction yields vs. pH for asparagine acid and glutamine acid.

Fig. 4 is a chromatogram showing the resolution of 17 amino acids as fluorescent labeled diastereomeric carbamates performed with reversed phase liquid chromatography.

Fig. 5 is a chromatogram showing the resolution of racemic metoprolol.

Fig. 6 is a chromatogram showing derivatization and resolution of commercially available "optically pure" L-glutamine.

## Description of the Invention

The invention is based on the rapid reaction of chloroformates with different amines to form stable carbamates during Schotten-Baumann conditions. The reaction is performed in one step within seconds at room temperature. In a certain pH range the reaction is selective to amino functions and the only formed by-product is the corresponding alcohol. The reagent excess is easy to handle whereby the interference in the following separation step is omitted.

The 9-fluorenylmethylchloroformate reagent (FMOC) is fluorescent with excitation/emission wavelengths of 270/315 nm. Thus the formed products are selectively detected at this wavelengths combination. This property together with the selective reaction lead to that very complex mixtures e.g. body fluids can be handled without any clean-up or pretreatment procedure.

The invention is to synthesize an optically active reagent which can be used in the same way as the achiral FMOC-reagent, with its many advantages, so to form diastereomers with racemic amino-containing compounds. The diastereomers should be feasible to resolve on a conventional separation system typically reversed phase HPLC, the most common and effective LC separation system for trace analysis and preparative separation. Making the reagent optically active is certainly not a guarantee for obtaining resolvable diastereomers. The introduction of an asymmetric carbon atom must be carried out at a position so that the reagent properties are maintained.

The invention was to create chirality of the FMOC reagent by introducing an asymmetric carbon atom at the 9-fluorenyl-1 position. This position is strategic while it is close to the formed amide function as well as to the rigid cyclic fluorene moiety. This type of compound has not been described before in the literature. It should be pointed out that there are still no general rules to design chiral reagents amenable for successful resolution of diastereomers. Therefore it is not possible to make predictions. Thus optical resolution is still based on trial and error experiments. However, surprisingly the 9-fluorenyl-1-ethylchloroformate reagent (FLEC) yields diastereomers with most amines resolvable on reversed phase LC columns. Furthermore the (+)-1-(9-fluorenyl)ethylchloroformate gave diastereomers of the rare D-amino acids which were separated prior to the main L-form. The properties of the new reagent were maintained compared to achiral FMOC reagent. As a conclusion a new reagent is described which will be

very practical and useful for the determination of optically active amino containing compounds in complex mixtures and for the preparative resolution of amines.

The reaction of FLEC with amino acids proceeds as shown in Fig. 1. The reagent also reacts with water to yield the corresponding alcohol as a hydrolysis product.

Synthesis of (+)-1-(9-fluorenyl)ethyl chloroformate (FLEC).
Synthesis of 1-(9-fluorenyl)ethanol: To a solution of 8.3 g fluorene in 100 ml of dry ether 31 ml BuLi (1.6 M in hexane) was added. The mixture was refluxed for 30 min. and then cooled in an ice bath. To the resulting mixture a solution of 2.8 ml acetaldehyde in 40 ml dry ether was added during 15 min. and then refluxed for 1 h. Water (100 ml) was added and the ether layer collected in a separatory funnel, dried ($MgSO_4$) and evaporated. The product was further purified by flash-chromatography and recrystallized from ligroin (b.p. 80—110°C) to give white needles, m.p. 101—103°C, mass spectrum $M^+$m/e 210.15, $^1H$ NMR($CDCl_3$), 0.87 (d, 3H), 1.70 (S, 1H), 4.09 (d, 1H), 4.35—4.65 (m, 1H), 7.15—7.85 (m, 8H).

Optical resolution of 1-(9-fluorenyl)ethanol: To a solution of 4.0 g 1-(9-fluorenyl)ethanol in 25 ml anhydrous pyridine, an equimolar amount of (−)-camphanic acid chloride (4.12 g) was added, and the mixture was stirred at room temperature for 3 h. The solution was poured into ice-water and extracted with $CH_2Cl_2$. The $CH_2Cl_2$-layer was washed with dilute hydrochloric acid, dried ($MgSO_4$) and evaporated to dryness. The crude ester was dissolved in 200 ml MeOH and cooled to −15°C. Deposited crystals (fraction A) were crystallized twice from the same solvent giving 1.0 g of the less soluble diastereomeric ester in pure form, (m.p. 159—160°C), (racemate mp. 152°C). The optical purity of the diastereomeric ester was checked on a chiral stationary phase ((−)-dinitrobenzoylphenylglycine coupled to aminopropylsilica, Pirkle's phase) and it was 99%.

Hydrolysis of the ester: To a solution of the optically pure diastereomeric ester (1.0 g) in 50 ml dry ether, 0.8 g $LiAlH_4$ was added. The mixture was stirred at room temperature for 1 h giving the alcohol after work up, m.p. 91—93°C (decomp.).

(+)-1-(9-fluorenyl)ethyl chloroformate: To a solution of phosgene (0.8, 8.1 mmol) in 15 ml of dry toluene cooled to 0°C, a solution of optically pure alcohol (0.44 g, 2.1 mmol) and triethylamine (0.30 ml, 2.1 mmol) in 20 ml dry toluene, was added dropwise. After addition was completed, stirring was continued for 2 h at 0°C. The triethylaminehydrochloride was then removed by filtration and the filtrate was concentrated at reduced pressure giving an oil.
$[\alpha]^{25} = +67.9°$ ($CH_2Cl_2$, C=1)
$[\alpha]^{25}_{578} = +70.5°$ ($CH_2Cl_2$, C=1)
$^1H$ NMR ($CDCl_3$): 0.76 (d, 3H), 4.30 (d, 1H), 5.47—5.75 (m, 1H), 7.18—7.75 (m, 8H)

| | | | |
|---|---|---|---|
| Anal. calcd for $C_{16}H_{13}O_2Cl$: | C, 70.46; | H, 4.80; | Cl, 13.00 |
| Found: | C, 70.64; | H, 4.80; | Cl, 13.13 |

Experimental conditions

Solvents and reagents: Acetonitrile, tetrahydrofurane and acetone were purchased from Rathburn (Walkerburn, U.K.). The amino acid standards and the FMOC reagent were obtained from Sigma (St. Louis, MO, U.S.A.). The elution buffer was made of acetic acid in double distilled water (3 mL/L), titrated to the appropriate pH with sodium hydroxide. The FLEC—Cl reagent was dissolved in acetonitrile:acetone (1:3) and had a concentration of 15 mmol/L. The reaction buffer was made of boric acid (1M) and the pH was adjusted with sodium hydroxide.
Derivatization: Sample (0.4 mL) and buffer (0.1 mL, pH) are mixed in a 3 ml reaction vial. The reagent (0.5 mL) is added and allowed to react. After 1 minute the vial is almost filled with pentane and the reaction mixture is extracted to remove excess reagent. The extraction is repeated twice, and then the aqueous phase is ready for injection.
Reaction rate: The rate of the reaction of the amino acids with the FLEC reagent is shown in Fig. 2 and was determined by derivatization of the amino acids (one at a time) in the usual manner with the reagent at pH 8.03 (sample plus buffer). After a certain time interval the reaction was stopped by addition of acetic acid, and the excess of the FLEC reagent was removed by pentane extractions. The amount that remained unreacted was determined by precolumn derivatization with o-phtalaldehyde/mercaptoethanol, followed by liquid chromatography. The results were compared with a solution treated in the same way, but without the FLEC reagent (original amount of amino acid), from which the conversion to a FLEC-derivative could be calculated. Each point is a mean of two measurements.

The effect of pH on the reaction rates of aspartic acid and glutamic acid is shown in Fig. 3 and was carried out in the same way as described above with 1 minute reaction time. Measurements were made at pH 8.01, 8.46, 9.01, 9.47 and 10.23. The pH was measured in the reaction solution after the addition of the buffer.

The reactive reaction rates of the FLEC and the FMOC reagents with valine, glutamic acid, proline and lysine were determined by comparison of the yield obtained when a standard amino acid solution was

derivatized with each of the reagents, with the results when a mixed FLEC/FMOC reagent was employed. The derivatives were separated with chromatography and the peak areas compared.

In fig. 4 is shown the resolution of 17 amino acids as fluorescent labelled diastereomeric carbamates performed with reversed phase liquid chromatography.

Experimental conditions were: The FLEC reagent is dissolved in acetonitrile/acetone (1/4), 15mM. The reaction buffer is a borate buffer, 1M, pH 6.5.

0.4 ml sample and 0.1 ml borate buffer are mixed. To this solution is added 0.5 ml of the reagent. After 1 min. reaction time the mixture is extracted 3 times with pentane. The pentane extracts are discarded and the aqueous phase is ready for injection. When derivatizing relatively hydrophobic amines not having an acid group, the extraction step is omitted and the excess reagent is removed by reaction with a hydrophilic amine, typically hydroxyproline or hydrazine.

TABLE I

| Gradient elution: | Time (min) | % AcN | %THF | %Buffer |
|---|---|---|---|---|
| | 0 | 8 | 17 | 75 |
| | 8 | 8 | 17 | 75 |
| | 22 | 0 | 30 | 70 |
| | 70 | 0 | 50 | 50 |

Separation conditions (amino acids):

Column: 150 × 4.6 mm packed with Spherisorb octyl material, d=3μm. Elution buffer: Acetic acid 3 promille, pH 4.35. Flow rate: 0.3 ml/min.

In table II below are stated the k'-values and resolution factors, α-values for some amino-containing pharmaceuticals.

TABLE II

| Amine | k'1 | k'2 | α |
|---|---|---|---|
| Metoprolol | 6.8 | 7.5 | 1.1 (1) |
| Tokainid | 9.4 | 10 | 1.06 (2) |
| Norephedrine | 5.03 | 5.28 | 1.05 (3) |

Column: 250 × 4.6 mm, packed with Spherisorb® octyl material, d=5μm. Flow rate: 1.2 ml/min.
Elution conditions: (1) 60% Acetonitrile, 40% water
(2) 50% Acetonitrile, 50% water
(3) 55% THF (tetrahydrofurane), 45% water

In Fig. 5 is shown the resolution of a racemic solution of metoprolol, a cardioselective β-adrenoceptor antagonist (β-blocker), derivatized with a racemic FLEC reagent. Column: 4.6 × 250 mm. packed with 5μm octyl material (Spherisorb): Eluent: 60% acetonitrile, 40% water.

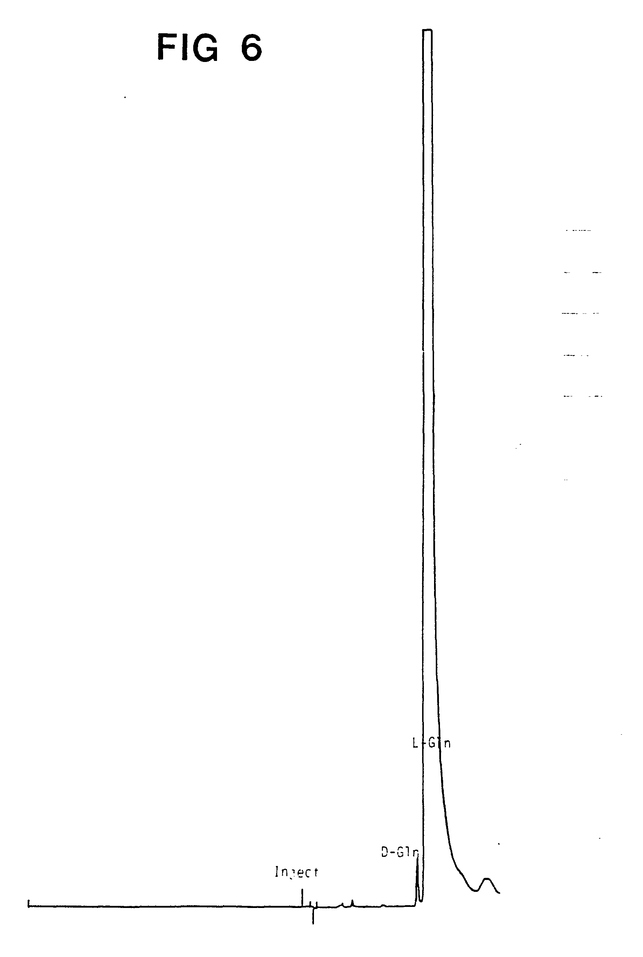

In Fig. 6 is shown the derivatization and resolution of commercially available "optically pure" L-glutamine to determine the combined effect of optical purity of the amino acid standard, the optical purity of the reagent and the racemization of the derivatization. L-glutamine (1mmol/l) derivatized with FLEC. The D-glutamine peak amounts to 0.6 promille of the L-glutamine, determined by standard addition of a small amount of D-glutamine (2μmol/l).

In Table III below are stated the k' and resolution factors, α-values, for the common peptide amino acids separated on reversed phase column.

TABLE III

| Amino acid | $k'_1$ | $k'_2$ | α | |
|---|---|---|---|---|
| Aspargine | 1.34 | 1.46 | 1.09 | I |
| Glutamine | 1.29 | 1.46 | 1.13 | |
| Serine | 2.01 | 2.09 | 1.04 | |
| Aspartic acid | 2.37 | 2.54 | 1.07 | |
| Threonine | 2.94 | 3.31 | 1.13 | |
| Glutamic acid | 3.26 | 3.69 | 1.13 | |
| Arginine | 3.54 | 4.03 | 1.14 | |
| Alanine | 5.63 | 6.20 | 1.10 | |
| Proline | 5.51 | 5.51 | 1.00 | — |
| Tyrosine | 3.29 | 3.91 | 1.19 | II |
| Methionine | 3.57 | 4.29 | 1.20 | |
| Phenylalanine | 4.83 | 6.09 | 1.26 | |
| Valine | 5.00 | 5.91 | 1.18 | |
| Isoleucine | 8.17 | 9.69 | 1.19 | |
| Leucine | 8.46 | 10.20 | 1.21 | — |
| Histidine | 3.37 | 4.77 | 1.42 | III |
| Ornithine | 5.37 | 6.69 | 1.25 | |
| Lysine | 7.23 | 8.37 | 1.16 | — |

Eluent composition:
I 35% THF, 65% Acetic acid buffer (3%, pH 4.35)
II 40% THF, 60%
III 45% THF, 55%
Column: 4.6 × 250 mm, packed with 5 μm octyl material (Spherisorb)

As indicated above reagent excess can be removed by different methods. One method, preferably used when separating amines, is to react reagent excess with an amine yielding a product which does not interfere during the separation with the compounds to be determined. The amine as well as the derivative should be water-soluble. Examples of such amines are hydroxyproline and hydrazine.

Another method, preferably used when separating amino acids or amino sugars forming hydrophilic amino derivatives, is to extract reagent excess with a solvent of low polarity, e.g. pentane or hexane.

The reagent according to the present invention is also applicable for preparative resolution of amino-containing racemic mixtures. After separation of the derivatives by HPLC the amino-containing compounds are regained by conventional methods, such as acid or basic hydrolysis or by hydrogenolysis.

In table IV below a comparison of current precolumn derivatizing agents and FLEC is made which illustrates the superiority of the FLEC-reagent.

TABLE IV

| | FLEC | PITC GITC | OPA | Dansyl Dabsyl |
|---|---|---|---|---|
| Primary and secondary amino compounds | Yes | Yes | No | Yes |
| Automated | Yes | No | Yes | No |
| Stable derivatives | Yes | No | No | No |
| MDQ | 100—200 femtomoles | 1—100 picomoles | 100—200 femtomoles | 100—200 femtomoles |
| Major interferences | No | No | No | |

Although 1-(9-fluorenyl)ethyl chloroformate is the only reagent described by way of examples there are a number of structural analogues that may be used as well. Methyl as R in the general formula in claim 1 could be substituted by trifluoromethyl or a higher alkyl group. Instead of chlorine X could be bromine, an azide group or a succinimidyl group, which have been tested for the FMOC-reagent and found to be good leaving groups.

## Claims

1. A method for the resolution and the determination of compounds having an amino function in aqueous and non-aqueous samples, characterized in adding an optically active reagent of the formula:

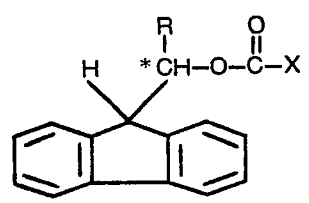

wherein X is a halogen, an azide group or a succinimidyl group and wherein R is an alkyl group or a trifluoromethyl group, to a sample containing compounds having a primary and/or secondary amino function so as to derivatize the amino function of the compounds and form diastereomeric carbamate derivatives which are then separated and determined by high performance liquid chromatography.

2. A method as claimed in claim 1, characterized in that R is a methyl group.

3. A method as claimed in claim 1 or 2, characterized in that X is chlorine.

4. A method as claimed in claim 1, characterized in that excess reagent is reacted with an amine yielding a product which does not interfere during the separation and determination of the derivatives.

5. A method as claimed in clailm 1, characterized in that excess reagent is extracted away with a solvent of low polarity, e.g. pentane or hexane, when hydrophilic amino derivatives are formed, e.g. amino sugars and amino acids.

6. A method as claimed in claim 1, characterized in that the compounds having an amino function are regained after separation and determination.

7. An optically active compound of the formula

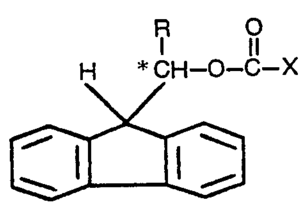

wherein X is halogen, an azide group or a succinimidyl group and wherein R is an alkyl group or a trifluoromethyl group.

8. A compound as claimed in claim 7, characterized in that X is chlorine.

9. A compound as claimed in claim 7 or 8, characterized in that R is a methyl group.

10. A compound according to claim 7 which compound is (+)-1-(9-fluorenyl)ethyl chloroformate.

**Patentansprüche**

1. Verfahren zur Trennung Bestimmung von Verbindungen mit einer Aminofunktion in wäßrigen und nicht-wäßrigen Proben, dadurch gekennzeichnet, daß man ein optisch aktives Reagens der Formel

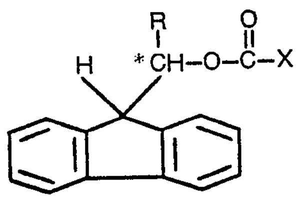

worin X für Halogen, eine Azidgruppe oder eine Succinimidylgruppe steht, und worin R eine Alkylgruppe oder eine Trifluormethylgruppe bedeutet, zu einer Probe zugibt, die Verbindungen mit einer primären und/ oder sekundären Aminofunktion enthält, um die Aminofunktion der Verbindungen zu derivatisieren und diastereomere Carbamatderivate zu bilden, die dann getrennt und durch Hochleistungs-Flüssigkeits-Chromatographie bestimmt werden.

2. Verfahren gemäß Anspruch 1, dadurch gekennzeichnet, daß R eine Methylgruppe ist.

3. Verfahren gemäß Anspruch 1 oder 2, dadurch gekennzeichnet, daß X ein Chloratom ist.

4. Verfahren gemäß Anspruch 1, dadurch gekennzeichnet, daß überschüssiges Reagens mit einem Amin unter Erzielung eines Produkts umgesetzt wird, das bei der Trennung und Bestimmung der Derivate nicht stört.

5. Verfahren gemäß Anspruch 1, dadurch gekennzeichnet, daß überschüssiges Reagens mit einem Lösungsmittel niedriger Polarität, z.B. Pentan oder Hexan, herausextrahiert wird, wenn hydrophile Aminoderivate, z.B. Aminozucker und Aminosäuren, gebildet werden.

6. Verfahren gemäß Anspruch 1, dadurch gekennzeichnet, daß die Verbindungen mit einer Aminofunktion nach der Trennung und Bestimmung wiedergewonnen werden.

7. Optisch aktive Verbindung der Formel

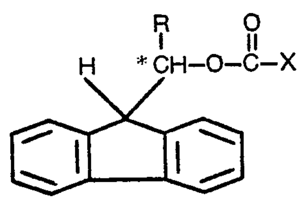

worin X für Halogen, eine Azidgruppe oder eine Succinimidylgruppe steht, und worin R eine Alkylgruppe oder Trifluormethylgruppe ist.

8. Verbindung gemäß Anspruch 7, dadurch gekennzeichnet, daß X Chlor bedeutet.

9. Verbindung gemäß Anspruch 7 oder 8, dadurch gekennzeichnet, daß R eine Methylgruppe ist.

10. Als Verbindung gemäß Anspruch 7 das (+)-1-(9-Fluorenyl)-ethylchloroformiat.

**Revendications**

1. Procédé de dédoublement et de détermination de composés ayant une fonction amino dans des échantillons aqueux et non aqueux, caractérisé par les étapes consistant à additionner un réactif optiquement actif de formule:

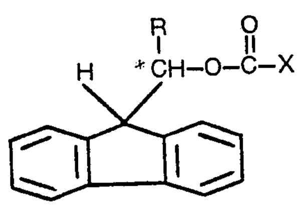

où X est un halogène, un groupe azide ou un groupe succinimidyle, et où R est un groupe alkyle ou un groupe trifluorométhyle, à un échantillon contenant des composés ayant une fonction amine primaire et/ou secondaire, de façon à former un dérivé de la fonction amine des composés et former des dérivés carbamates diastéréoisomères, qui sont ensuite séparés et déterminés par chromatographie en phase liquide de haute performance.

2. Procédé selon la revendication 1, caractérisé en ce que R est un groupe méthyle.

3. Procédé selon la revendication 1 ou 2, caractérisé en ce que X est le chlore.

4. Procédé selon la revendication 1, caractérisé en ce que l'on fait réagir le réactif en excés avec une amine, conduisant à un produit qui n'interfère pas avec la séparation et la détermination des dérivés.

5. Procédé selon la revendication 1, caractérisé en ce que l'on extrait le réactif en excès avec un solvant de faible polarité, par exemple le pentane ou l'hexane, lorsqu'il se forme des dérivés amino hydrophiles, par exemple des sucres aminés et des acides aminés.

6. Procédé selon la revendication 1, caractérisé en ce que les composés ayant une fonction amine sont récupérés après la séparation et la détermination.

7. Composé optiquement actif de formule

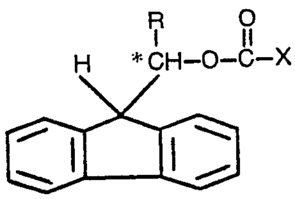

où X est un halogène, un groupe azide ou un groupe succinimidyle, et où R est un groupe alkyle ou un groupe trifluorométhyle.

8. Composé selon la revendication 7, caractérisé en ce que X est le chlore.

9. Composé selon la revendication 7 ou 8, caractérisé en ce que R est un groupe méthyle.

10. Composé selon la revendication 7, qui est le chloroformiate de (+)-1-(9-fluorényl)éthyle.

FIG 1

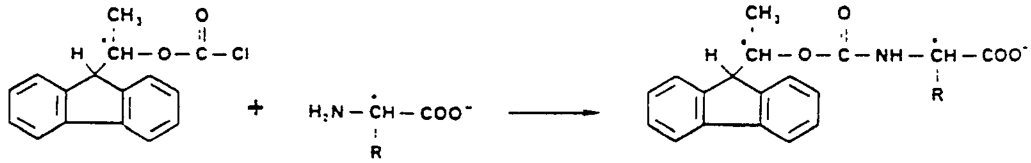

FIG 2

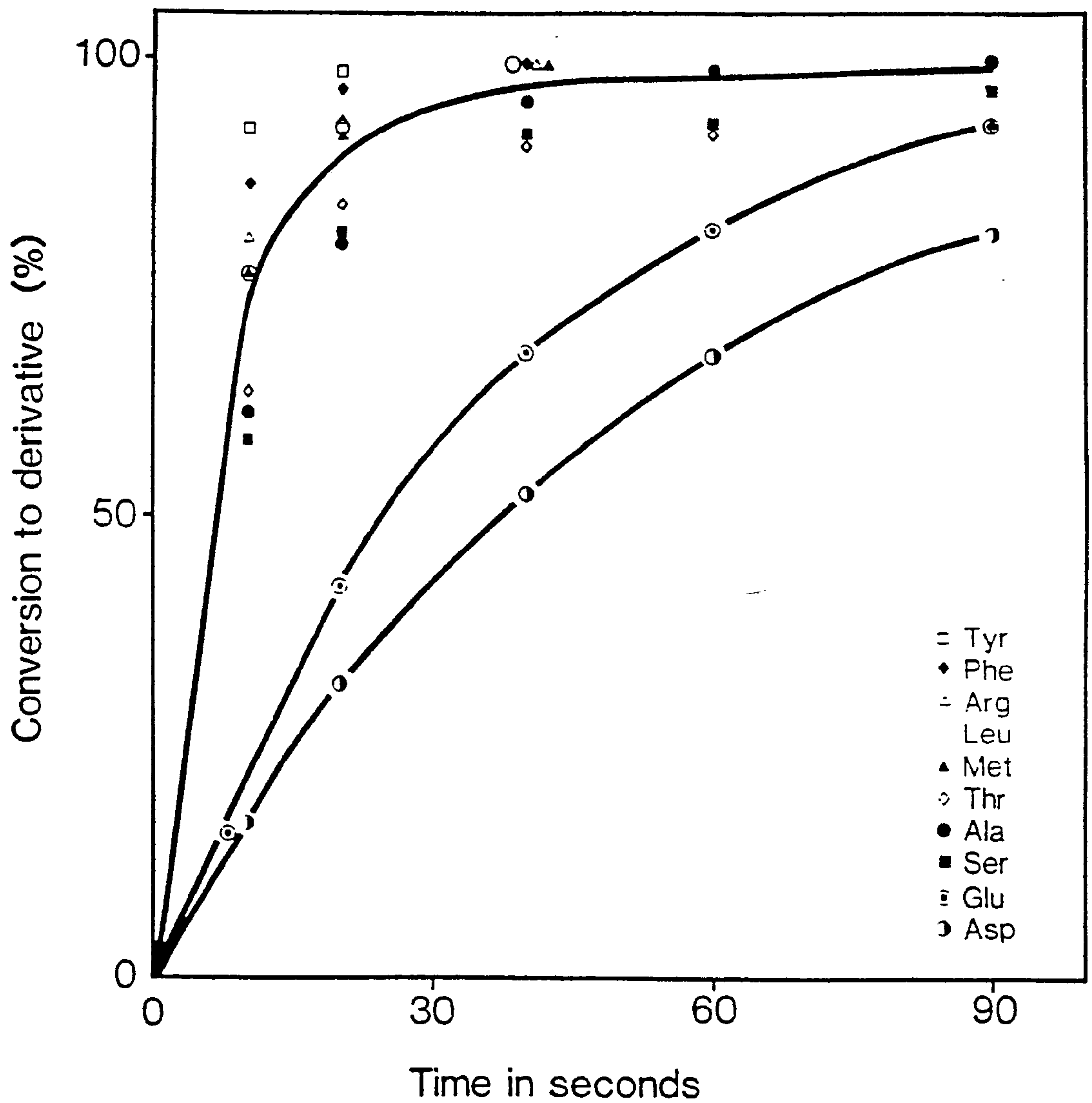
100
50
0
0
30
60
90
Conversion to derivative (%)
Time in seconds
Tyr
Phe
Arg
Leu
Met
Thr
Ala
Ser
Glu
Asp

FIG 3

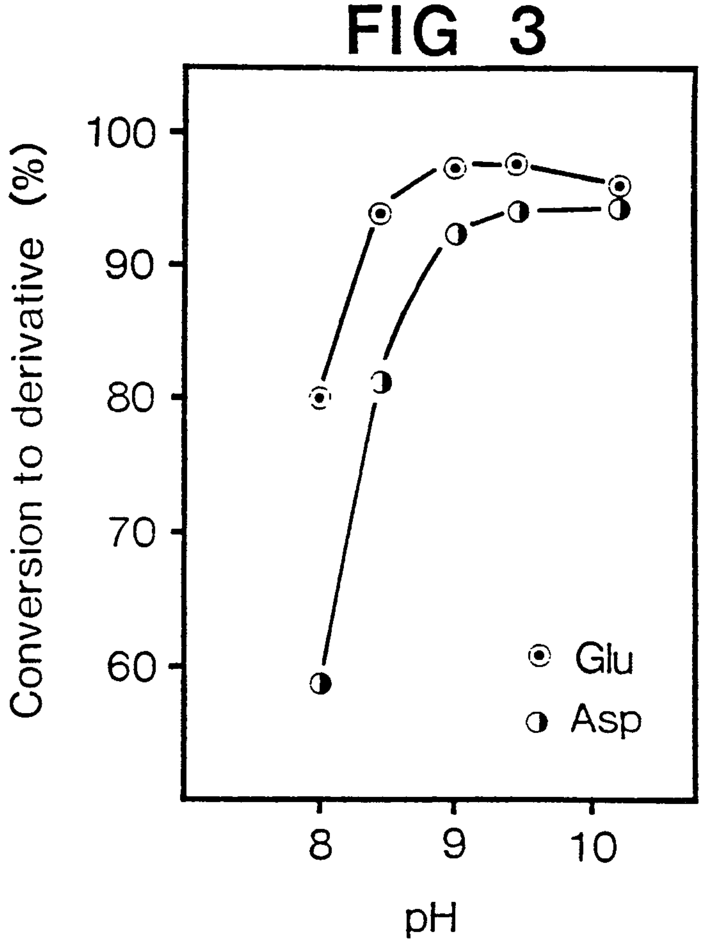

FIG 4

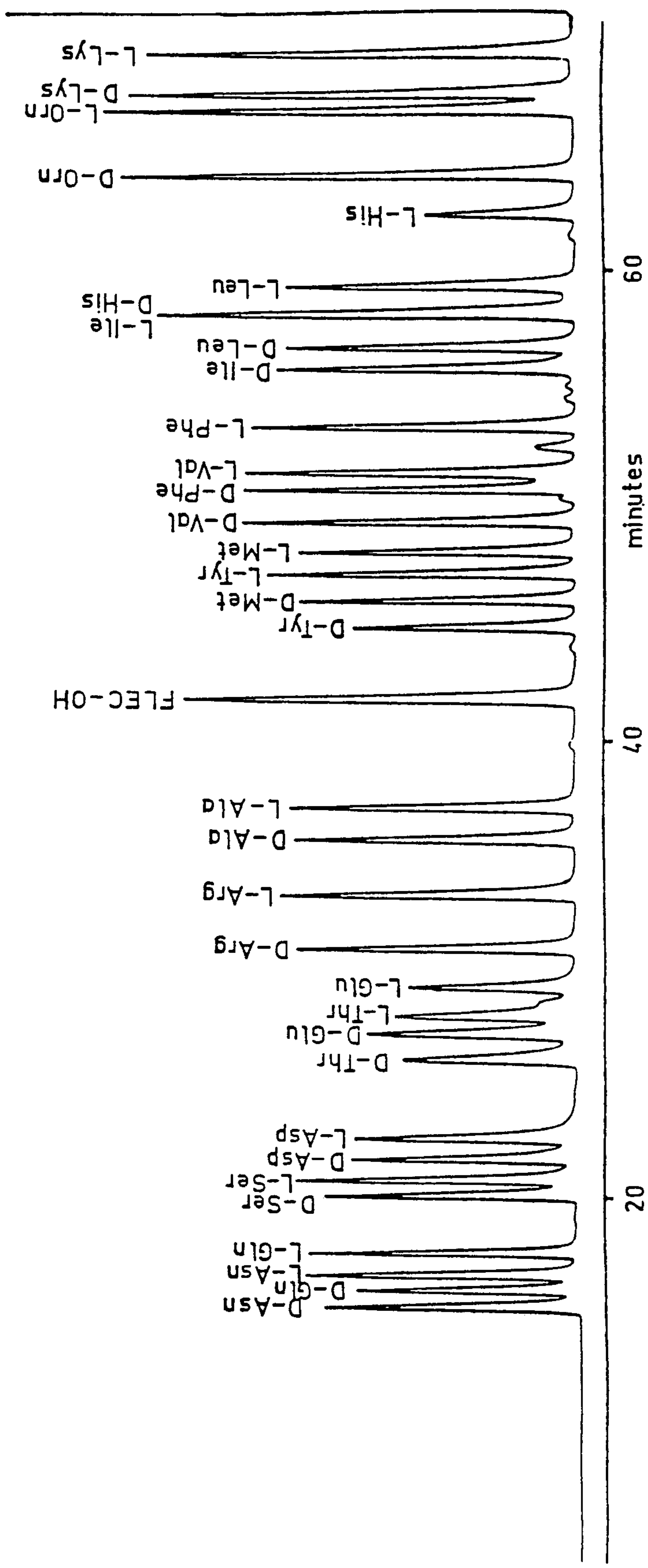
D-Asn
D-Gln
L-Asn
L-Gln
D-Ser
L-Ser
D-Asp
L-Asp
D-Thr
D-Glu
L-Thr
L-Glu
D-Arg
L-Arg
D-Ala
L-Ala
FLEC-OH
D-Tyr
D-Met
L-Tyr
L-Met
D-Val
D-Phe
L-Val
L-Phe
D-Ile
D-Leu
L-Ile
D-His
L-Leu
L-His
D-Orn
L-Orn
D-Lys
L-Lys
20
40
60
minutes

FIG 5

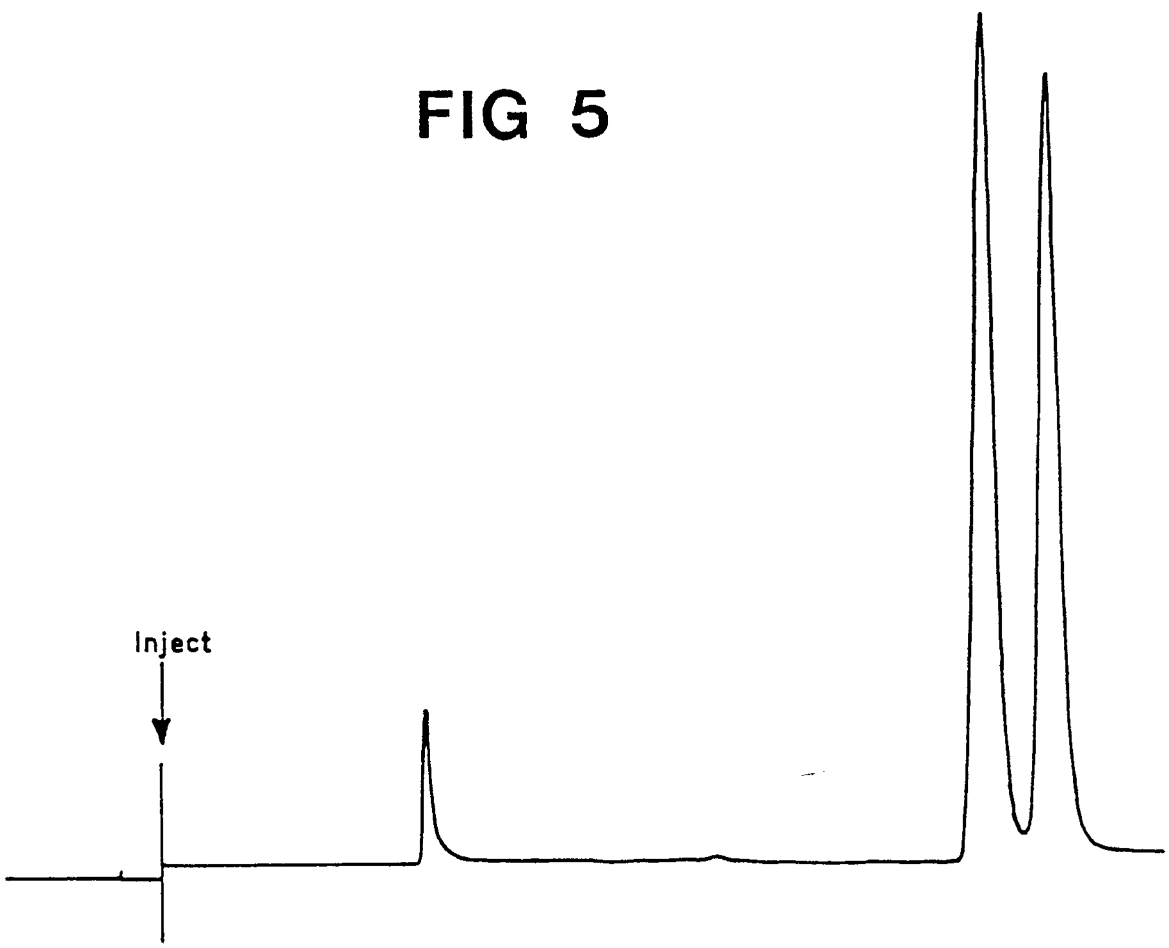

FIG 6